(19) **Europäisches Patentamt / European Patent Office / Office européen des brevets**

(11) **EP 1 112 485 B1**

(12) # EUROPEAN PATENT SPECIFICATION

(45) Date of publication and mention
of the grant of the patent:
**30.12.2009 Bulletin 2009/53**

(51) Int Cl.:
*G01N 22/04* (2006.01)    *G01N 22/00* (2006.01)

(21) Application number: **99931444.6**

(22) Date of filing: **11.06.1999**

(86) International application number:
**PCT/IN1999/000024**

(87) International publication number:
**WO 2000/077501 (21.12.2000 Gazette 2000/51)**

(54) **AN APPARATUS AND METHOD FOR MEASURING AND MONITORING COMPLEX PERMITTIVITY OF MATERIALS**

VERFAHREN UND VORRICHTUNG ZUR MESSUNG UND ÜBERWACHUNG DER KOMPLEXEN PERMITTIVITÄT VON MATERIALIEN

APPAREIL ET PROCEDE PERMETTANT DE MESURER ET DE SURVEILLER LA PERMITTIVITE COMPLEXE DE MATERIAUX

(84) Designated Contracting States:
**DE GB**

(43) Date of publication of application:
**04.07.2001 Bulletin 2001/27**

(73) Proprietor: **Joshi, Kalpana
Northampton NN45 5BU (GB)**

(72) Inventor: **Joshi, Kalpana
Northampton NN45 5BU (GB)**

(74) Representative: **Raynor, Simon Mark et al
Urquhart-Dykes & Lord LLP
Midsummer House
413 Midsummer Boulevard
Central Milton Keynes MK9 3BN (GB)**

(56) References cited:
EP-A1- 0 971 227    GB-A- 2 277 803
US-A- 3 510 764    US-A- 3 942 107
US-A- 4 829 233    US-A- 5 334 941

- **PATENT ABSTRACTS OF JAPAN & JP 08 220 160 A (MURATA) 30 August 1996**
- **SOVIET PATENTS ABSTRACTS Section SX, Week 9248, Derwent Publications Ltd., London, GB; Class 501, AN 1992-397227/48, XP002945101 & SU 1 707 570 A1 (OBNINSK) 23 January 1992**
- **SOVIET PATENTS ABSTRACTS Section SX, Week 9032, Derwent Publications Ltd., London, GB; Class 501, AN 1990-245396/32, XP002945102 & SU 1 539 681 A (AS USSR APPL PHYS) 30 January 1990**

- **SOVIET INVENTIONS ILLUSTRATED Section EL, Week 8701, Derwent Publications Ltd., London, GB; Class 501, AN 1987-006062/01, XP002945103 & SU 1 231 474 A (BERLINYUA) 15 May 1984**
- **EBBE NYFORS AND PERTTI VAINIKAINEN: "INDUSTRIAL MICROWAVE SENSORS" 1989, XP002334964 ARTECH HOUSE, Norwood, MA, United States of America ISBN: 0-89006-397-4**
- **K.K.JOSHI, M.ABEGAONKAR, R.N.KAREKAR, R.C.AIYER: "MICROSTRIP RING RESONATOR AS A MOISTURE SENSOR FOR WHEAT GRAINS" IEEE MTT-S DIGEST, TH3F-49, 1997, pages 1679-1682, XP010228373**
- **R.A.YOGI, S.A.GANGAL, R.C.AIYER, R.N.KAREKAR: "Microwave ring resonator as a novel bio-material moisture sensor" SENSORS AND ACTUATORS B, vol. 50, 1998, pages 38-44, XP004142101**
- **M.P.ABEGAONKAR, R.N.KAREKAR, R.C.AIYER: "A microwave microstrip ring resonator as a moisture sensor for biomaterials: application to wheat grains" MEAS. SCI. TECHNOL., vol. 10, 1999, pages 195-200, XP000887471 UK**
- **JOSHI K K; POLLARD R D; POSTOYALKO V: "Conductor, dielectric and radiation losses in multilayer microstrip interconnection with dielectric cover for high performance packaging" ELECTRICAL PERFORMANCE OF ELECTRONIC PACKAGING,, 1995, pages 67-69, XP010195166 Portland, OR, USA**

EP 1 112 485 B1

**(Cont. next page)**

- JOSHI K K; POLLARD R D; POSTOYALKO V: "Microstrip with dielectric overlay: variational analysis and validation" IEE PROCEEDINGS: MICROWAVES, ANTENNAS AND PROPAGATION, vol. 141, no. 2, 4 January 1994 (1994-01-04), pages 138-140, IEE, STEVENAGE, HERTS, GB
- KAMAL SARABANDI: "MICROSTRIP RING RESONATOR FOR SOIL MOISTURE MEASUREMENTS" IEEE TRANSACTIONS ON GEOSCIENCE AND REMOTE SENSING, vol. 35, no. 5, September 1997 (1997-09), pages 1223-1231,
- TASUO ITOH: "A new Method for Measuring Properties of Dielectric Materials using a Microstrip Cavity" IEEE TRANSACTIONS ON MICROWAVE THEORY AND TECHNIQUES, 1974, pages 572-576,
- JOSHI K K, POLLARD R D: "Microstrip resonator technique for non-destructive moisture/ permittivity measurement" IEEE MTT S INTERNATIONAL MICROWAVE SYMPOSIUM DIGEST, vol. 4, 13 June 1999 (1999-06-13), - 19 June 1999 (1999-06-19) pages 1863-1866, Anaheim, CA, USA
- EIKICHI YAMASHITA: "Variational Method for the Analysis of Microstrip-Like Transmission Lines" IEEE TRANSACTIONS ON MICROWAVE THEORY AND TECHNIQUES, vol. MTT-16, no. 8, 1968, pages 529-535,
- P LOWES, S SCOTT, E KOROLIEWICZ, A SAMBELL: "Quasistatic capacitance and frequency-dependent effective permittivity of multilayer microstrip patch antennas" IEE PROC-MICROW. ANTENNAS PROPAG., vol. 145, no. 1, February 1998 (1998-02), pages 75-79, XP006011230
- C BERNOU, D REBIERE, J PISTRE: "Microwave sensors: a new sensing principle. Application to humidity detection" SENSORS AND ACTUATORS B, vol. 68, 2000, pages 88-93,
- I J BAHL, S S STUCHLY: "Analysis of a Microstrip Covered with a Lossy Dielectric" IEEE TRANSACTIONS ON MICROWAVE THEORY AND TECHNIQUES, vol. MTT-28, no. 2, February 1980 (1980-02), pages 104-109,

**Description**

<u>FIELD OF INVENTION</u>

This invention relates to an apparatus and method for measuring and monitoring complex permittivity of materials.

<u>PRIOR ART</u>

Microstrip and microstrip type resonators described are efficient devices for measuring complex permittivity of materials at microwave frequencies as disclosed by Flemming U.S. Patent No. 4,829,233; by Heath U.S. Patent No. 3,510,764; and by Gerhard U.S. Patent No.3,942,107 and by King U.S. Patent No.5,334,941. Flemming describes a method in which a resonator is mounted on the copper-backed substrate. A resonator is weekly coupled to a microwave feed source and to a microwave detector so that the resonator Q factor is unaffected by the impedance's of the source or the detector. When the test dielectric is placed near the resonator, the electromagnetic fields near the resonator are coupled to the material under test so as to affect the resonator frequency of resonance as well as Q factor.

The resonant frequency and Q factor measurements are done in the transmission mode. Further the methods for modulating source or resonant frequency are disclosed which avoids the need for the swept source.

Heath uses a half wavelength microstrip resonator, which is tightly sandwiched between two sheets of sample test material. These sheets of sample material are clamped in a special fixture. The microstrip resonator is loosely capacitively coupled to the microstrip feed line, which passes near one end of the resonator normal to the resonator length. The dielectric constant is determined from the measurement of resonant frequency and Q factor for the transmission between sensor's two input and output ports. As the special cutting and positioning of thin sheets of the sample material is required; Heath's method is not in situ or non-destructive. King relates to the microwave resonator reflection sensor for complex permittivity measurement in situ. The microstrip resonator is fed from the ground plane side through a slot.

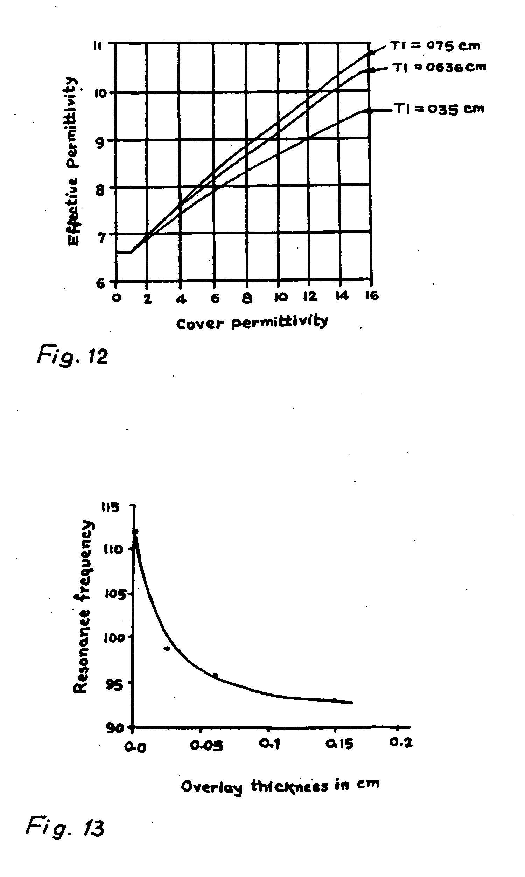

Microwave power is coupled to the slot through a coaxial line or a microstrip. The material under test is kept in contact with the sensor. The resonant frequency and input power coupling factor is measured at the resonant frequency. The real and imaginary parts of permittivity ($\varepsilon'$ and $\varepsilon''$) or the conductivity ($\sigma$) are determined from the resonant frequency and coupling coefficient data using approximate closed form expressions. King uses bottom fed resonators which claims to be a major modification but this leads to complicated assembly and unstable mounting compared to the side-coupled resonator. The approximation in the basic expression of capacitance of the cross section of the sensor leads to the serious inaccuracies in the results. The closed form expressions need use of standards for calibration to evaluate constants. King depends upon empirical or analytical calibration. Evaluation of constants before the installation and commissioning of the sensor is essential. The starting equation of effective capacitance of King is based on the assumption that the fringing fields constant is same for all thickness of sample. Our analysis (fig.12) shows that the resonant frequency varies with the permittivity as well as material thickness. King is limited to the measurement of infinitely thick samples only. The empirical calibration techniques of King leads to errors if the thickness of the sample is different than the calibration standard. King claims the in situ sensor but it is not possible to perform in situ calibrations in most situations, as the chamber cannot be filled with the calibration liquid or solid. King leads to errors in the calculated and true data due to calibrations using standards. The same is true for the analytical calibrations as the actual surface irregularities and air gaps between resonator and the solid sample contribute to the errors in measurements. It is essential to validate analytical technique with the standard data. Dispersion in microstrip with dielectric overlay also plays an important part in errors. None of the empirical calibration techniques using closed form expressions account for dispersion. The shifts in the resonant frequency can be larger than 1 GHz at the material permittivities of 10(fig.13). Hence dispersion affects accuracy at first decimal place of permittivity. Both Flemming and Heath involve transmission from one input port to an output port. Both ports are loosely and capacitively coupled to the intervening resonator by capacitive coupling. King relates only to the critically coupled reflection one port sensor. King relates to the samples of very large size compared to the sensor. Gerhard relates to the measurement real part of the dielectric constant only. The samples under consideration are thin sheets of dielectric materials. Gabelich's measures dielectric homogeneity at 100 KHz to 2MHz for the use of dielectric substrates for CTS-ESA radar antennas on Barium STrontium Titanate (BST). The C band or microwave permittivity is co-related to low frequency permittivity even though it is not accurate enough. Gabelich's measures only real part of permittivity.

US patent no 5686841 Nov 11,1997, Stolarczyk et. al teach the use of patch antenna to detect presence of ice, water and/or antifreeze mixtures on wings of the aircraft or roads. A single frequency is fed to the antenna which is one of the arms of the bridge circuit. This frequency is varied until admittance of the antenna approaches zero. The object of the Stolarczyk's invention is not to accurately determine complex or real permittivity but to only detect formation of ice. The frequency resolution of the frequency variation is very low, of the order.of 2.4MHz.

Article T. Itoh: "A New Method for Measuring Properties of Dielectric Materials Using a Microstrip Cavity", IEEE TRANSACTIONS ON MICROWAVE THEORY AND TECHNIQUES, May 1974, p.572 discloses a method for nondestructively measuring both the real part of the permittivity and tanδ of a sample by means of a straight line microstrip resonator of the transmission type or the reflection type. First a reference material, which may be conveniently chosen to be the same as the substrate, is placed on the microstrip resonator and both the resonant frequency $\omega_1$ and the quality factor $Q_1$ are measured. Then the measurement is repeated by replacing the reference material with the unknown material to obtain $\omega_2$ and $Q_2$. The conductance $G_1$ per unit length and the capacitance $C_1$ per unit length with reference material are calculated numerically from material parameters of the substrate and reference material using two variational expressions. The capacitance $C_2$ per unit length with the unknown sample is determined from $C_1$ and the measured resonance frequencies $\omega_1$ and $\omega_2$ using the equation $\omega_2/\omega_1=(C_1/C_2)^{0.5}$ neglecting the influence of the sample at the ends of the straight line resonator. The conductance $G_2$ with the unknown sample is determined from $\omega_1/Q_1 - \omega_2/Q_2 = G_1/C_1 - G_2/C_2$. The real part of the permittivity of the sample is determined numerically from $C_2$ using one of said variational expressions. Tanδ of the sample is determined numerically from $G_2$ and the real part of the permittivity of the sample using the other one of said variational expressions.

Article K. Sarabandi and E. S. Li: "Microstrip Ring Resonator for Soil Moisture Measurements", IEEE TRANSACTIONS ON GEOSCIENCE AND REMOTE SENSING, Vol.35, No.5, Sep.1997 discloses a method for measuring the real and imaginary parts of the permittivity $\varepsilon_2 = \varepsilon'_2 - j\varepsilon''_2$ of a dielectric medium using a microstrip ring resonator. First the resonant frequencies $f^l$ and $f^u$ of the loaded (resonator in contact with the dielectric medium) and unloaded (resonator in free space) resonators are measured. The substrate is assumed to be lossless. When the radiation losses of the loaded resonator and unloaded resonator are the same or the radiation loss is negligible compared to the conductor and substrate losses, equation $1/Q_m=1/Q_u+1/Q_d$ can be used to determine the quality factor $Q_d$ due to the dielectric loss from the quality factor $Q_u$ of the unloaded resonator and the measured quality factor $Q_m$. The real part $\varepsilon'_{eff}{}^l$ and the imaginary part $\varepsilon''_{eff}{}^l$ of the effective permittivity $\varepsilon_{eff}$ of the loaded resonator is then determined in terms of measured $f^u/f^l$ and $Q_d$ and calculated $\varepsilon_{eff}{}^u$ using the equations $f^u/f^l=Re(\sqrt{\varepsilon_{eff}})/\sqrt{\varepsilon_{eff}}{}^u$ and $\varepsilon''_{eff}{}^l=\varepsilon'_{eff}{}^l/Q_d$ .Once $\varepsilon'_{eff}{}^l$ and $\varepsilon''_{eff}{}^l$ are thus obtained, the loaded line capacitance $C^l$ and loaded line conductance $G^l$ are determined from further equations. The last step in the inversion algorithm is the computation of $\varepsilon'_2$ and $\varepsilon''_2$ of the cover sample from the measured $C^l$ and $G^l$. This is done numerically based on a quasistatic formulation.

Article I.J.Bahl and S.S.Stuchly: "Analysis of a Microstrip Covered with a Lossy Dielectric", IEEE TRANSACTIONS ON MICROWAVE THEORY AND TECHNIQUES, Vol. MTT-28, No.2, Feb. 1980 discloses a method for determining the effective permittivity of a cover sample of straight line microstrip resonators. Two straight line resonators of different lengths were used to cancel the effect of the cover sample at both ends of the straight line resonators. This article discloses also the following closed form expression for the attenuation constant $\alpha_d$ due to dielectric loss only

$$\alpha_d = \frac{27.3}{\lambda_0 \sqrt{\varepsilon_e}} \left[ \left( \frac{\varepsilon_{e1} - 1}{\varepsilon_{r2} - 1} \right) \varepsilon_{r2} \tan \delta_2 + \left( \frac{\varepsilon_e - \varepsilon_{e1}}{\varepsilon_{r1} - 1} \right) \varepsilon_{r1} \tan \delta_1 \right]$$

wherein $\lambda_0$ is the free space wavelength, $\varepsilon_e$ is the effective permittivity of the covered Microstrip, $\varepsilon_{e1}$ is the effective permittivity of the uncovered microstrip, $\varepsilon_{r1}$ , $\varepsilon_{r2}$ and $\tan\delta_1$, $\tan\delta_2$ are the relative permittivities and loss tangents of the substrate and the cover sample respectively.

OBJECTS OF THE INVENTION

A primary object of this invention is to propose a process and apparatus for the measurement and monitoring of complex permittivity and conductivity of materials in situ which is accurate.

Another object of this invention is to propose a process and apparatus for the measurement and monitoring of complex permittivity and conductivity of materials in situ using automatic scalar or vector network analyzers or swept frequency generators and peak detectors that perform fast and accurate frequency and amptitude measurements.

Still another object of this invention is to propose a process and apparatus for the measurement and monitoring of complex permittivity and conductivity of materials in situ employing online numerical analysis software performing numerous iterations to arrive at convergence within few seconds with required accuracy like $\pm$ 0.1 or $\pm$ 0.01 in case of $\varepsilon$ and $\pm$ 0.0001 or $\pm$ 0.0005 in case of $\varepsilon$.

Yet another object of this invention is to propose a process and apparatus for the measurement and monitoring of complex permittivity and conductivity of materials in situ and wherein the determination of real and imaginary parts of permittivity takes place in a relatively short period.

A further object of this invention is to propose a process and apparatus for the measurement and monitoring of complex permittivity and conductivity of materials in situ and wherein the permittivity measurement does not depend on the first order approximations and simplistic closed form expressions involving unkown constants.

A still further object of this invention is to propose a process and apparatus for the measurement and monitoring of complex permittivity and conductivity of materials in situ and used for high frequency circuits boards, various bulk polymers and semiconductor materials.

Yet a further object of this invention is to propose a process and apparatus for the measurement and monitoring of complex permittivity and conductivity of materials in situ and wherein transmission type resonators may be used for samples of smaller size than the resonator.

DESCRIPTION OF THE INVENTION:

The invention provides a device and process for measuring and monitoring complex permittivity of materials for quality control, in situ and in the materials measurement laboratory. Material under test is kept as an overlay on microstrip, asymmetric stripline, co-planar waveguide, patch or a disc resonator. The resonator has its resonant frequency in the range of 0.5 to 20 GHz. The material is placed in contact with the top conductors of the circuits or with a finite air gap above the top conductor. The ground plane at the top may or may not be used. Fringing field from the top surface and the edges of the resonator passes through the material under test kept as an overlay dielectric. As the fields above the substrate pass through the material under test the effective permittivity of the resonator increases. The Q factor ($Q=\beta/2\alpha$) of the resonator changes due to change in the propagation constant ($\beta$) and attenuation constant ($\alpha$). Increase in the effective permittivity of a microstrip, asymmetic stripline, coplanar waveguide resonator, rejection filter, patch or a disc causes decrease in the resonant frequency of the resonator.

Q factor of the resonator decreases as the attenuation due to overlay adds to the total losses.

The invention envisages the use of transmission as well as reflection type resonators. The resonators are coupled to the source of microwave power using direct or gap coupling (capacitively or inductively) from the side of the microstrip conductor and not from the ground plane side. The measurements may be one port or two port depending upon the dimensions of the material under test. If a swept frequency source is used then a resonant dip may be observed in the reflection or transmission mode with the available instrument accuracy. Q factor is directly measured using half power frequencies and the resonant frequency. The unloaded Q measurement is preferred for the accurate measurement $\varepsilon$" or $\sigma$. If loaded Q is measured then it is necessary to calculate unloaded Q from loaded. Q factor. The resonant or half power frequencies is automatically measured and fed to the computer using data acquisition system and/or frequency tracking device. The dedicated computer program calculates real and imaginary parts of the complex permittivity or conductivity of the material under test. The program is based on the numerical analysis of a microstrip embedded in multiple dielectric layers, the material under test being the layer of unknown permittivity.

DESCRIPTION OF INVENTION WITH REFERENCE TO ACCOMPANYIND DRAWINGS

The invention will be further described with the help of accompanying drawings, in which

Fig.1 shows a block diagram of an instrument using scalar or vector Network analyzer

Fig.2 shows a diagram of an instrument using power meter or ratio meter

Fig.3 shows a block diagram of an instrument with the reflection type sensor and the test specimen

Fig.4 shows a schematic diagram of a direct coupled, reflection type sensor (microstrip atch antenna) and the test specimen

Fig.5 shows a schematic diagram of a direct coupled, reflection type microstrip sensor (quarter or half wavelength) and the test specimen

Fig.6 shows a schematic diagram of a gap coupled, transmission and/or reflection type sensor (quarter or half wavelength) and the test specimen

Fig.7. shows a cross sectional diagram of a transmission and/or reflection type microstrip sensor and the test specimen of finite height

Fig.8 shows a cross sectional diagram of a transmission and/or reflection type microstrip sensor and bulk test specimen of with a very large dimensions

Fig.9 shows a cross sectional diagram of a transmission and/or reflection type asymmetric stripline sensor and test specimen of finite height

Fig.10 shows a schematic diagram of a direct coupled, reflection type coplanar waveguide sensor (quarter or half wavelength) and the test specimen

Fig.11 shows a schematic diagram of a gap coupled, transmission type microstrip ring resonator sensor (one wavelength) and the test specimen

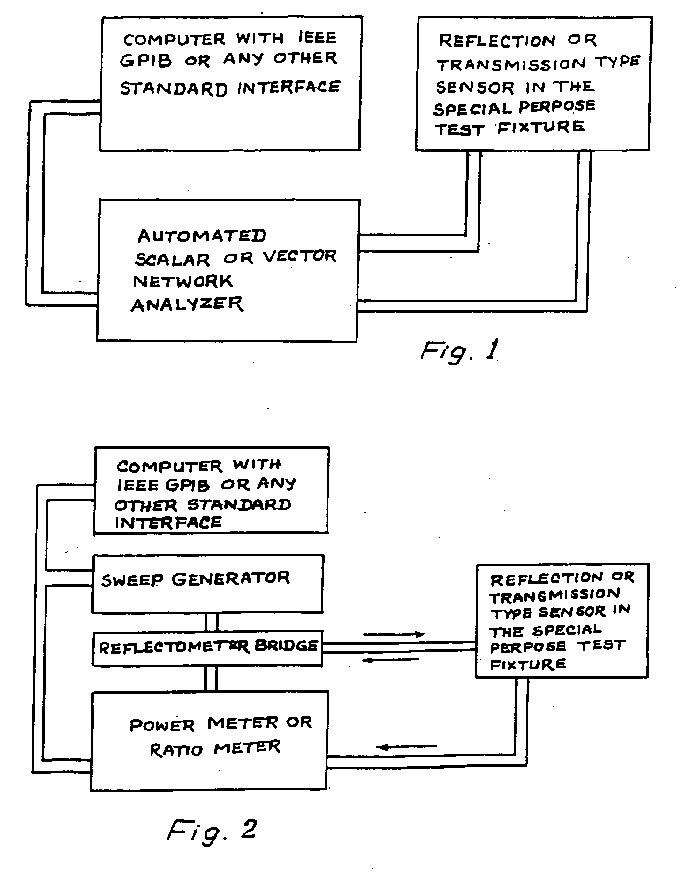

With reference to the drawings, Fig.1 and 2 are schematic diagrams showning an arrangement of the apparatus for the use in this invention. Fig.1 shows an instrument for the determination of complex permittivity of materials comprising:

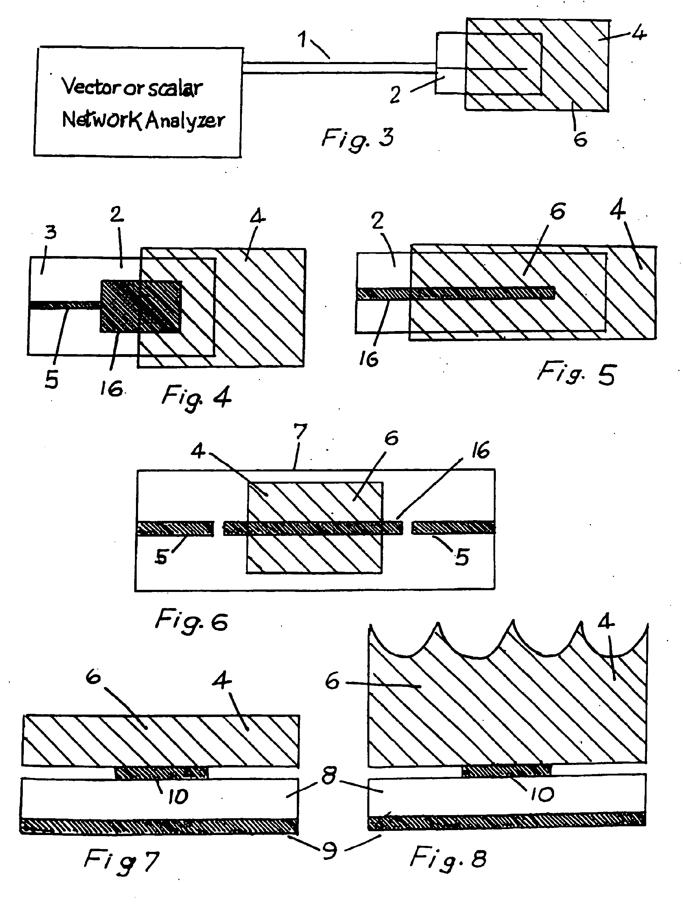

I. Automated scalar or vector network analyzer; II. reflection or transmission type microwave sensors of any type and as shown in Fig.3,4,5,6,7,8,9,10,11; III. A computer with IEEE GPIB or any other standard interface; IV. Data acquisition and postprocessing numerical analysis software for the determination of complex permittivity loaded on the computer.

Fig. 2 shows schamatic diagram of the instrument using I. sweep generator; II. reflectometer bridge or directional coupler; III. power meter or VSWR meter; IV. Computer with IEEE GPIB or any other standard interface; 5. reflection or transmission type microwave sensors 2 or 7 of any type shown in fig.3,4,5,6,7,8,9,10,.11. The network analyzer has a built in synthesized sweep generator which generates swept frequency output with the required frequency span. The frequency resolution of the sweep generator can be as high as 1 Hz. The output power level may be adjusted to the required value. The swept microwave power output of the sweep generator is coupled to the sensor circuit via a special purpose test fixture and a coaxial cable. If the sensor in the test fixture is of reflection type then a directional coupler or a reflectometer bridge is used to isolate reflected power and then it is coupled to the detector port. The reference signal is being fed externally in case of a scalar network analyzer. The network analyzer calculates ratio of reflection and/or transmitted parameters to the input power to the sensor for the range of swept frequencies. The reasonant frequency is displayed on the screen as a sharp peak is observed due to the storage of power in the resonator. The half power frequencies are observed on the screen with the help of automatic 3 dB search or manually. According to the block diagram of Fig.2, sweep generator generates swept signal and is fed to the sensor 2 or 7 through the reflectometer bridge. The test specimen 4 of unkown permittivity is kept as an overlay on the reflection sensor. Microwave power is coupled to the reflection sensor 2 or 7 through a coaxial cable using standard coaxial to microstrip transition like SMA connector. The reflectometer separates reflected signal from the outgoing signal and is measured by the power meter. The transmitted signal can be measured by the power meter directly. The resonance is detected by the displayed power output reading of the sensor at the particular frequency. The frequency resolution of the instrument and process is the resolution of the sweep generator which can be as high as 1 IIz.

Fig.3 shows a block diagram with an automated scalar or vector network analyzer; coaxial cable 1, refection type microwave sensor 2, test specimen 4 of unknown permittivity 6 kept as an overlay on the reflection sensor.

Fig.4 shows a schematic diagram of a microstrip patch antenna 16 as a reflection sensor 2 delineated on substrate 3 of known permittivity $\varepsilon$ 1, with the feed line 5 and the test material 4 kept as an overlay.

Fig.5 shows a schematic diagram of a microstrip quarter or half wavelength resonator as a reflection sensor 2 delineated on substrate 3 of known permittivity $\varepsilon 1$, with the feed line 5 and the test material 4 of unknown permittivity $6(\varepsilon)$ kept as an overlay.

Fig.6 shows a schematic diagram of a gap coupled half wavelength microstrip resonator as a transmission sensor 7 with the feed line 5 and the test material 4 of unknown permittivity $\varepsilon$ kept as an overlay.

Fig.7 shows a cross sectional diagram of a microstrip sensor conductor 10, ground plane 9, substrate 8 and the test specimen 4 of finite height and unknown permittivity $6(\varepsilon)$ kept as an overlay.

Fig.8 shows a cross sectional diagram of a microstrip sensor conductor 10, ground plane 9, substrate 8 and the test specimen 4 of finite height and unknown permittivity $6(\varepsilon)$ kept as an overlay. The bulk test specimen 4 is shown to be of very large dimensions.

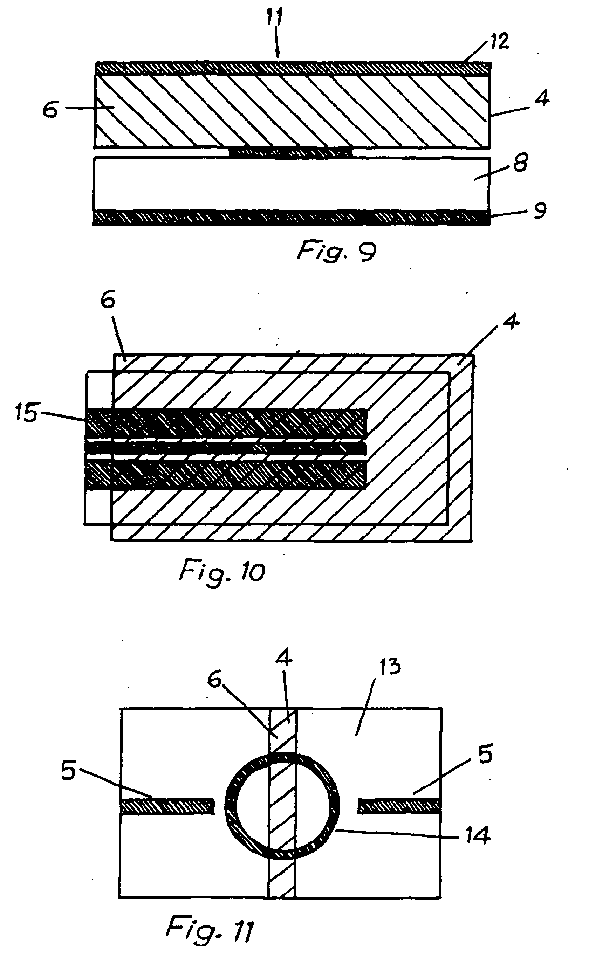

Fig.9 shows a cross sectional diagram of a transmission and/or reflection type asymmetric stripline sensor 11, with a microstrip sensor conductor 10, ground plane 9, substrate 8 and the test specimen 4 of finite height and unknown permittivity $6(\varepsilon)$ kept as an overlay and a top ground plane 12. Fig.10 shows a schematic diagram of a direct coupled, reflection type coplanar waveguide sensor 15(quarter or half wavelength) and the test specimen 4 unknown permittivity $6(\varepsilon)$ kept as an overlay.

Fig.11 shows a schematic diagram of a gap coupled, transmission type microstrip ring resonator sensor 14(one wavelength) and the test specimen 4 of smaller dimensions than the resonator length having unknown permittivity $6(\varepsilon)$ kept as an overlay at the 90 orientation with respect to the feed line 5.

Fig.12 shows results of the numerical software of the present invention simulating a reflection or transmission type microstrip sensor. The graph shows variation of effective permittivity with respect to cover permittivity and test material thickness when the test material completely overlaps the sensor. This graph indicates the difference between other inventions of using microstrip resonator sensors kept in the vicinity of test material. This invention does not use any approximate closed form expressions for the calibration of sensor but uses on-line numerical analysis which does not require use of known standards or empirical calibrations (like Flemming et al. US patent no.4,829,233 or King 5,334,941); but uses on-line computer software.

Fig.13 shows acomparison of experimental data with the simulation of frequency shift of 99.99% pure alumina kept as a overlay on a microstrip resonator.

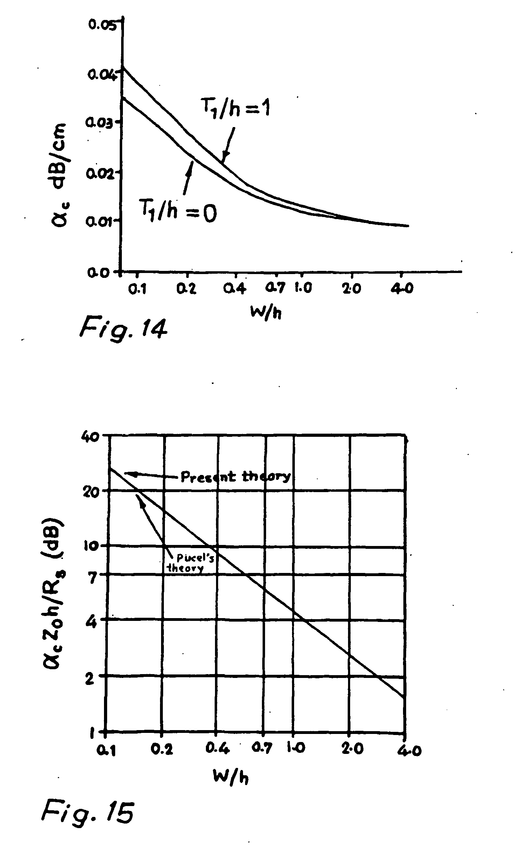

Fig.14 shows simulation of conductor attenuation with 99.99% pure alumina as a substrate and overlay on a microstrip resonator, where T is an overlay thickness and h is the substrate height

Fig.15 shows comparison of our simulation of conductor attenuation with Pucel's theory for open microstrip, where h is the substrate height.

An on line computer with data acquisition software and hardware is used. Resonant and half power frequency data from microwave test setup is manually fed to the computer through the interactive user friendly software for the postprocessing of data and determination of complex permittivity of specimen under test. A dedicated computer program computes complex permittivity using electromagnetic numerical analysis software analyzing resonator embedded (fig. 7,8) in multiple dielectric layers. The program takes less than three seconds on the IBM compatible computer with Pentium 350 $MH_z$ processor and three minutes on 80386 processor. The user can choose the required accuracy.

The material under test, 4 for which complex permittivity, 6 is to be measured is kept in direct contact of the resonator or keeping a gap of few microns from the resonator. A top ground plane, 12 is optional. The computer program needs to be fed the information on the type of resonator configuration as well as presence of top ground plane and thickness and length of the material under test. The test set up needs to be calibrated for acceptable connector assembly. The resonator is tested without the presence of material under test initially. The resonant frequency and half power frequency are measured and fed to the program. The sample or material under test, 4 is then placed over the resonator in the required orientation and the specified position. The sensor is placed in the reaction chamber or a drier or in the moving belt in case of flowing material without disturbing the connector assembly. The presence of dielectric material in the vicinity of the resonator shifts frequency of resonance due to the increase in the effective permittivity, $\varepsilon_{eff}$ of the resonator.

$$f_s/f_o = (\varepsilon_{eff o}/\varepsilon_{effs})^{0.5} \qquad (1)$$

Where subscripts "o" and "s" stand for open and with loaded sample.

At resonance electrical length of a microstrip resonator is an integral multiple of half wavelengths. The real part of effective permittivity ($\varepsilon_{eff}$) of an open resonator is determined by the software program by feeding width of the top conductor, substrate permittivity, substrate loss tangent and substrate permittivity. It is necessary to know these values most accurately as the errors and uncertainties in these values will reflect on the resultant values of permittivities of specimens kept as overlays. Hence utmost care is required in designing and fabricating the test vehicle. The $\varepsilon_{effs}$ thus determined is numerically postprocessed by the program to determine specimen real part of the permittivity $\varepsilon'_r$; the required accuracy may be fed to the interactive program at the maximum of $\pm 0.01$. The Quality factor, Q of the resonator changes due to the additional dielectric and conductor losses due to the overlay material. Our numerical analysis shows increase in the total loss due to dielectric cover (fig.15). The unloaded Q factor of a resonator is given by the standard reltion.

$$Q_{total} = \beta/2\alpha \qquad (2)$$

Where

$$\alpha_{total} = \alpha_{conductor} + \alpha_{dielectric} + \alpha_{radiation} \qquad (3)$$

where $\beta$ is a propagation constant and

$$Q_{total} = Q_{unloaded}$$

The software also calculates conductor loss for the microstrip with cover specimen. This is not same as an open microstrip conductor loss[7]. The program evaluates the dissipation factor of cover specimen from the given data using electromagnetic numerical analysis. The radiation loss can be neglected if desired.

$$\alpha_{total} - \alpha_{conductor} = \alpha_{dielectric} \alpha_{radiation} \qquad (4)$$

Neglecting radiation losses the program calculates contribution of cover dielectric to the dielectric loss and hence gives the value of dissipation factor tan of the sample

$$1/Q_{total} = 1/Q_c + 1/Q_d + 1/Q_r \qquad (5)$$

where

$Q_c$ = Quality factor due to conductor losses
$Q_d$ = Quality factor due to dielectric losses
$Q_d$ = Quality factor due to radiation losses

Let $Q_{t(open)}$ = Total Quality factor of an open micro-strip resonator
$Q_{t(cover)}$ = Total Quality factor of a covered microstrip resonator
$\alpha_{t(open)}$ = Total attenuation per unit length of an open microstrip resonator
$\alpha_{t(cover)}$ = Total attenuation per unit length of a covered microstrip resonator

$$\alpha_{t(cover)} = \alpha_{c(cover)} + \alpha_{d(open)} + \alpha_{d(cover)} + \alpha_{r(cover)} \qquad (6)$$

The numerical analysis indicates conductor loss $\alpha_{c(cover)}$ as well as $\alpha_d$ of a microstrip with dielectric cover is larger than the open microstrip. For low permittivity and lossy materials the increase in the conductor loss may be neglected

$$1/Q_{t(cover)} - 1/Q_{t(open)} = 1/Q_{d(cover)} \qquad (7)$$

Neglecting radiation losses. See also discussion of the article of Sarabandi above.

$$d = [27.3/(\lambda_0 \varepsilon_{effs})] [q_1 \varepsilon_1 \tan\delta_{(substrate)} + q_2 \varepsilon_2 \tan\delta_{(cover)}] \qquad (8)$$

where

$q_1$ = filling fraction due to substrate
$\varepsilon_1$ = real part of permittivity of substrate
$q_2$ = filling fraction due to dielectric cover
$\varepsilon_2$ = real part of permittivity of the cover

See also discussion of the article of Bahl and Stuchly above.

$$\alpha_{d(cover)} = [27.3 / (\lambda_0 \varepsilon_{effs})] \; [q_2 \varepsilon_2 \tan \delta_{(cover)}] \quad (9)$$

It can be noticed that determination of $\varepsilon''$ or $\tan \delta$ requires a value of $\varepsilon'$ of the test material. King (US patent no. 5334941) neglects dependence of $\varepsilon'$ on the effective capacitance and treats effective capacitance as a constant, which is certainly not the case. Therefore determination of $\varepsilon''$ has inherent inaccuracy in King's method.

This invention actually used a highly accurate value of $\varepsilon'$ in the determination of $\varepsilon''$ along with the numerically calculated conductor and dielectric losses.

$$1/Q_{d(cover)} = \pi / \alpha_{d(cover)} \lambda_g \quad (10)$$

$$\alpha_{d(cover)} = \pi Q_s / \lambda_g \quad (11)$$

From equations (9) and (11)

$$\tan \delta_{(cover)} = \pi Q_s (\varepsilon_{effs})^{1.5} / 27.3 \; q_2 \varepsilon_2 \quad (12)$$

where the program uses the value of 2 obtained numerically beforehand and

$$q^2 = (\varepsilon_{effs} - \varepsilon_{effo}) / (\varepsilon_1 - 1) \quad (13)$$

In case materials with very low values of $\tan \delta$ increase in the conductor loss due to increased effective permittivity needs to be substracted from the total loss. The computer program takes care of it from the determined value of $\tan \delta$. Hence the process is applicable to materials with high and low loss dielectrics including materials used for high frequency applications in microwave region.

Measurement of material samples that are smaller in dimensions than that of the resonator:

The invention provides a process of measurement of complex permittivity of samples that are smaller in length(fig. 6) and breadth(fig.11) than that of the resonator. The examples of such samples are single grain of wheat, coffee, rise, GaAs, diamond and other precious stones, various types of medicine capsules etc. The invention can be used for studying sample to sample variation in the complex permittivity of objects of the dimensions of few millimeters. The software will ask for the dimensions of the material sample under test and will follow the required subroutine. For a halfwave resonator

$$l_1 (\varepsilon_{eff1})^{0.5} + l_2 (\varepsilon_{eff2})^{0.5} = n \lambda_0 / 2 \quad (14)$$

where subscripts 1 and 2 stand for different materials overlaying the resonator.

If the sample is partially covering the resonator in the region 2 then $\varepsilon_{eff1}$ assumes the value of effective permittivity for the open microstrip and $l_1$ is the length of the open region of the resonator. $\varepsilon_{eff2}$ is the unknown value of effective permittivity of the overlaid microstrip at the frequency of operation. Equation holds for both real and imaginary parts of $\varepsilon_{eff}$. The effect of dispersion needs to be taken into account. In the present invention software is taking care of dispersion. The unknown effective permittivity is determined using the equation (15) by the software.

It is possible to use this technique at the manufacturing site to monitor chemical reactions involving insulating or low conductivity materials and quality of materials like poly tetra fluro ethylene (PTFE) and other polymer, rubbers and foams. The instrument and the process may be used at the various stages of manufacturing or curing or processing of polymer, organic, food or agricultural products to ensure the final quality. The process and instrument may be used to detect voids, porosity, cracks, and non-uniformity of permittivity or density of the material.

The microstrip resonator used as a test vehicle may be pre-calibrated before the measurement starts. It may also be possible to use calibration standard overlays to define system uncertainties. After establishing acceptable connector assembly the connector and fixture repeatability does not affect sample to sample measurements. This is due to the fact that input and output cables and connections to the resonator and/or test vehicle are tested before the sample is loaded and are unaltered thereafter during measurement. It is not necessary to connect the disconnect the the test vehicle if next sample is to be measured. This is a significant circuit boards and materials like IPC-TM-650, no. 2.5.5.5.1, and the document of International Packaging Commission.

The test vehicle design may include care for avoiding loading error so that loading error is avoided with confidence. This is a distinct advantage as the loading error uncertainties remain to be tackled at the time of measurements in method IPC-TM-650, no. 2.5.5.5.1. The loading time for the specimen may be of a few seconds. The response of a network analyzer is within fraction of a second. Determination of permittivity with the computer takes few seconds. Therefore the measurements may be ver fast for multiple samples. The accepted methods require either fabrication of microstrip resonators on the sample under test or need a complicated assembly clamping which may take longer for each sample measurement.

The present invention exploits maximum resolution of frequency source and detector of Network analyzers which is quoted to be $1H_z$ by Hewlett Packard for its vector Network analyzers. Patch antenna of either rectangular or disc shaped is used to determine real dielectric constant with the accuracy of $\pm 0.015$ and tan to the accuracy of $\pm 0.0001$ or more. Single antenna or any other type of resonator is used to measure complex permittivity with the help of online numerical analysis program.

There are many differences and uses of the present invention in comparison with the inventions of Health, Flemming, Gerhard, Gabelich and King. In particular variety of sample dimensions can be accommodated from 3 mm (fig.6,11) e.g. wheat or rice grain or pallet or tablet; to very large dimensions in length and breadth (fig.4,5,8,10). Depth of the sample may be as small as 0.1 mm (fig.6,7,8) to few meters depth (Fig.4,5,8,10,11). The present invention can also be used for the measurement of complex permittivity (fig. 7,8,10) and dielectric homogeneity in the sheets of dielectric materials. The test material needs to be moved relative to the sensor or multiple sensors may be employed in the production process. The present invention uses on-line numerical analysis software which has been validated for variety of materials of known permittivities which include ceramic and semiconductor substrates like 99% alumina and semi-insulating GaAs, high frequency circuit board materials like PTFE and wheat grains. The present invention provides on-line or of line numerial analysis software which gives results of complex permittivity directly from the resonant frequency and Q factor data acquired from the scalar or vector network analyzer or the microwave test equipment. The thickness, length and breadth of the sample need to be fed to the program along with the data on sensor dimensions in the set up routine.

**Claims**

1.   A device for the measurement of complex permittivity of dielectric materials in solid, liquid or semisolid state comprising

(a) a signal source selected from microwave sweep oscillator and variable signal source of microwave power
(b) resonator sensor selected from transmission and reflection type
(c) a means for providing orientation to the sample
(d) a microwave power measurement device selected from a set of a detector, power meter, network analyzer, VSWR meter for the measurement of resonant frequency and Q factor
(e) means for determining effective permittivity using equation

$$f_o{}^2/f_s{}^2 = \varepsilon_{effs}/\varepsilon_{effo}$$

(with $f_o$, $f_s$, $\varepsilon_{effo}$, $\varepsilon_{effs}$ being the resonant frequencies and effective permittivities of the open resonator and the resonator loaded with sample)
and using an online numerical analysis software determining real part of the sample permittivity $\varepsilon_2$ from the effective permittivity $\varepsilon_{effs}$ iteratively until acquiring specified accuracy (e.g. 0.01) and further determining imaginary part of the sample permittivity using the Q values $Q_{t(open)}$, $Q_{t(cover)}$ of open resonator and resonator loaded with sample using equations

$$\tan\delta_{(cover)} = \pi Q_s (\varepsilon_{effs})^{1.5}/27.3 \; q_2\varepsilon_2$$

$$1/Q_{t(cover)} - 1/Q_{t(open)} = 1/Q_{d(cover)}$$

$$q_2 = (\varepsilon_{effs} - \varepsilon_{eff0})/(\varepsilon_1 - 1)$$

(with $\varepsilon_1$ being the real part of the permittivity of the substrate).

2. A process of using the device of claim 1 for the measurement of complex permittivity.

**Patentansprüche**

1. Vorrichtung zum Messen der komplexen Permittivität von dielektrischen Materialien in festem, flüssigem oder halbfestem Zustand, umfassend

(a) eine Signalquelle, die aus Mikrowellen-Kippschwingungsoszillator und variabler Signalquelle von Mikrowellenenergie ausgesucht ist
(b) Resonatorsensor, der aus Transmissions- und Reflexionstyp ausgewählt ist
(c) ein Mittel zum Orientieren der Probe
(d) eine Mikrowellenenergiemessvorrichtung, die aus einem Satz aus einem Detektor, Leistungsmesser, Netzwerkanalysator, Welligkeitsfaktor-(VSWR)-Messgerät zum Messen der Resonanzfrequenz und des Q-Faktors ausgewählt ist
(e) Mittel zum Bestimmen der effektiven Permittivität unter Verwendung der folgenden Gleichung

$$f_0^2 / f_s^2 = \varepsilon_{effs} / \varepsilon_{eff0}$$

(wobei $f_0$, $f_s$, $\varepsilon_{eff0}$, $\varepsilon effs$ die Resonanzfrequenzen und die effektiven Dielektrizitätskonstanten des offenen Resonators und des mit der Probe beladenen Resonators sind.)
und Software zur online numerischen Analyse verwendet, mit der der Realteil der Proben-Permittivität $\varepsilon_2$ aus der effektiven Permittivität $\varepsilon_{effs}$ iterativ bestimmt wird, bis eine vorbestimmte Genauigkeit (bspw. 0,01) erreicht wird, und ferner der Imaginärteil der Proben-Permittivität unter Verwendung der Q-Werte $Q_{t(open)}$, $Q_{t(cover)}$ des offenen Resonators und des Resonators, der mit der Probe beladen ist, mit Hilfe der folgenden Gleichungen bestimmt wird

$$\tan\delta_{(cover)} = \Pi Q_s (\varepsilon_{effs})^{1.5} \Big/ 27.3 \quad q_2\varepsilon_2$$

$$\frac{1}{Q_{t(cover)}} - \frac{1}{Q_{t(open)}} = \frac{1}{Q_{d(cover)}}$$

$$q_2 = (\varepsilon_{effs} - \varepsilon_{eff0})/(\varepsilon_1 - 1)$$

(wobei $\varepsilon_1$ der Realteil der Permittivität des Substrats ist.)

**2.** Prozess zur Verwendung der Vorrichtung nach Anspruch 1 zum Messen der komplexen Permittivität.

**Revendications**

**1.** Dispositif destiné à mesurer la permittivité complexe de matériaux diélectriques dans un état solide, liquide ou semi-solide comprenant :

> (a) une source de signaux sélectionnée parmi un oscillateur à relaxation hyperfréquence et une source de signaux variables d'énergie hyperfréquence ;
> (b) un capteur à résonateur de type sélectionné parmi un type à transmission et un type à réflexion ;
> (c) des moyens destinés à fournir une orientation à l'échantillon ;
> (d) un dispositif de mesure d'énergie hyperfréquence sélectionné dans le groupe constitué par un détecteur, un dispositif de mesure de puissance, un analyseur de réseau, un dispositif de mesure du rapport d'onde stationnaire (VSWR) pour la mesure de la fréquence de résonance et du facteur Q ;
> (e) des moyens destinés à déterminer la permittivité effective à l'aide de l'équation :

$$f_o^2 / f_s^2 = \varepsilon_{effs} / \varepsilon_{effo}$$

> ($f_o$, $f_s$, $\varepsilon_{eefo}$, $\varepsilon_{effs}$ étant les fréquences de résonance et les permittivités effectives du résonateur ouvert et du résonateur chargé avec l'échantillon) ;
> et en utilisant un logiciel d'analyse numérique en ligne qui détermine la partie réelle de la permittivité de l'échantillon $\varepsilon_2$ à partir de la permittivité effective $\varepsilon_{effs}$ de manière itérative jusqu'à obtenir la précision spécifiée (par exemple 0,01) et qui détermine en outre la partie imaginaire de la permittivité de l'échantillon en utilisant les valeurs de Q, $Q_{t(open)}$ et $Q_{t(cover)}$, du résonateur ouvert et du résonateur chargé avec l'échantillon à l'aide des équations :

$$\tan\delta_{(cover)} = \pi \, Q_s \, (\varepsilon_{effs})^{1,5} / 27,3 \, q_2 \, \varepsilon_2$$

$$1 / Q_{t(cover)} - 1 / Q_{t(open)} = 1 / Q_{d(cover)}$$

$$q_2 = (\varepsilon_{effs} - \varepsilon_{effo}) / (\varepsilon_1 - 1)$$

> ($\varepsilon_1$ étant la partie réelle de la permittivité du substrat).

**2.** Procédé destiné à utiliser le dispositif de la revendication 1 pour la mesure d'une permittivité complexe.

COMPUTER WITH IEEE
GPIB OR ANY OTHER
STANDARD INTERFACE

REFLECTION OR
TRANSMISSION TYPE
SENSOR IN THE
SPECIAL PERPOSE
TEST FIXTURE

AUTOMATED
SCALAR OR VECTOR
NETWORK
ANALYZER

*Fig. 1*

COMPUTER WITH
IEEE GPIB OR ANY
OTHER STANDARD
INTERFACE

SWEEP GENERATOR

REFLECTOMETER BRIDGE

REFLECTION OR
TRANSMISSION
TYPE SENSOR IN
THE SPECIAL
PERPOSE TEST
FIXTURE

POWER METER OR
RATIO METER

*Fig. 2*

Vector or scalar Network Analyzer

Fig. 3

Fig. 4

Fig. 5

Fig. 6

Fig 7

Fig. 8

Fig. 9

Fig. 10

Fig. 11

Fig. 12

Fig. 13

Fig. 14

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4829233 A, Flemming
- US 3510764 A, Heath
- US 3942107 A, Gerhard
- US 5334941 A, King
- US 5686841 A, Stolarczyk

### Non-patent literature cited in the description

- **T. Itoh.** A New Method for Measuring Properties of Dielectric Materials Using a Microstrip Cavity. *IEEE TRANSACTIONS ON MICROWAVE THEORY AND TECHNIQUES,* May 1974, 572
- **K. Sarabandi ; E. S. Li.** Microstrip Ring Resonator for Soil Moisture Measurements. *IEEE TRANSACTIONS ON GEOSCIENCE AND REMOTE SENSING,* September 1997, vol. 35 (5
- **I.J.Bahl ; S.S.Stuchly.** Analysis of a Microstrip Covered with a Lossy Dielectric. *IEEE TRANSACTIONS ON MICROWAVE THEORY AND TECHNIQUES,* February 1980, vol. MTT-28 (2